# EUROPÄISCHE PATENTSCHRIFT

(11) **EP 0 479 878 B1**
(45) Veröffentlichungstag und Bekanntmachung des Hinweises auf die Patenterteilung: **05.10.1994**
(21) Anmeldenummer: 90910689.0
(22) Anmeldetag: 25.06.1990
(51) Int. Cl.: C07C 49/83, C07C 45/64

(54) **VERFAHREN ZUR HERSTELLUNG VON 1,4-BIS-(4-HYDROXYBENZOYL)-BENZOL**
PROCESS FOR PREPARING 1,4-BIS-(4-HYDROXYBENZOYL)-BENZENE
PROCEDE DE PRODUCTION DE 1,4-BIS-(4-HYDROBENZOYL)-BENZENE

(30) Priorität: 30.06.1989 DE 3921449
(43) Veröffentlichungstag der Anmeldung: 15.04.1992
(73) Patentinhaber: HOECHST AKTIENGESELLSCHAFT, 65926 Frankfurt am Main (DE)
(72) Erfinder: HACKENBRUCH, Joachim, D-6095 Ginsheim (DE); PAPENFUHS, Theodor, D-6000 Frankfurt am Main (DE)
(86) Internationale Anmeldenummer: EP9001002
(87) Internationale Veröffentlichungsnummer: WO9100256

(56) Entgegenhaltungen:
- EP-A- 0 094 347
- DE-C- 865 595
- CHEMICAL ABSTRACTS, Band 80, 1974, Columbus, Ohio, USA; M. I. FARBEROV et al, "p-Bis(p-chloro-, -hydroxy-, and aminobenzoyl)benzene", S. 348, Zusammenfassung Nr. 82351d; SU-A-405 859

## Beschreibung

Die vorliegende Erfindung betrifft ein verbessertes Verfahren zur Herstellung von 1,4-Bis-(4-hydroxybenzoyl)-benzol durch Umsetzung von 1,4-Bis-(4-halogenbenzoyl)-benzol mit Alkalimetall- oder Erdalkalimetallhydroxiden in verschiedenen Lösungsmitteln bei erhöhten Temperaturen.

1,4-Bis-(4-hydroxybenzoyl)-benzol ist eine wichtige Monomerverbindung zur Herstellung technischer Kunststoffe und kann durch Reaktion mit 1,4-Halogenaromaten zu Polyetherketonen umgesetzt werden. Bisherige Herstellungsmethoden sind die Fries-Verschiebung von Terephthalsäurediphenylester (J.A.C.S. 1938, 2284; EP 75 390), die Entalkylierung von 1,4-Bis-(4-methoxybenzoyl)-benzol mit HBr (Doroshenko, Vysokomol. Soedin 8(10), 1787-92, 1966) sowie die nucleophile Substitutionsreaktion an 1,4-Bis-(4-chlorbenzoyl)-benzol mit Natronlauge in Wasser in Gegenwart von Kupfersalzen (SU 405 859). Diesen bekannten Herstellungsmethoden ist gemein, daß sie unter Verwendung gesundheitlich bedenklicher und zudem kostenspieliger Verbindungen bei hoher Umweltbelastung verlaufen.

Es wurde nun gefunden, daß der nucleophile Austausch der Hydroxylgruppe durch ein Halogenatom im Unterschied zum Verfahren, der SU-PS 405 859 ohne Kupfer in Gegenwart eines Lösungsmittels möglich ist und daß dieses Lösungsmittel nach beendeter Reaktion durch Destillation abgetrennt und in die nächste Umsetzung zurückgeführt werden kann.

Gegenstand der Erfindung ist somit ein gegenüber dem Stand der Technik verbessertes Verfahren zur Herstellung von 1,4-Bis-(4-hydroxybenzoyl)-benzol der Formel (I) 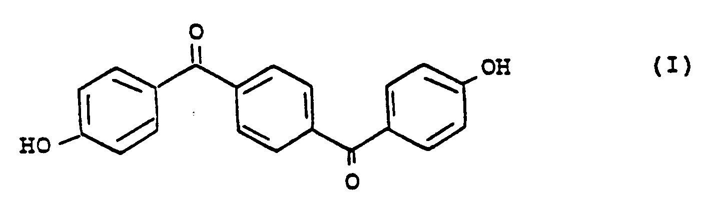
indem man 1,4-Bis-(4-halogenbenzoyl)-benzol der Formel (II) 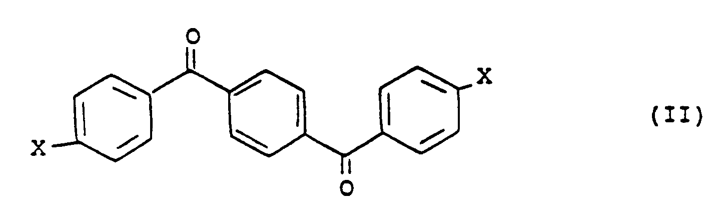
in welcher X ein Fluor-, Chlor- oder Bromatom bedeutet, mit einem Alkalimetallhydroxid, vorzugsweise Lithium-, Natrium-oder Kaliumhydroxid, oder mit einem Erdalkalimetallhydroxid, vorzugsweise Magnesium- oder Calciumhydroxid, oder mit Mischungen aus den vorstehend genannten Hydroxiden, in einem gegenüber den Reaktionskomponenten bei den Reaktionsbedingungen inerten organischen Lösungsmittel oder in einem Zweiphasensystem aus Wasser und einem inerten organischen Lösungsmitel bei Temperaturen von etwa 100° C bis etwa 250° C, vorzugsweise etwa 150° C bis etwa 220° C, ggfs. in Gegenwart eines Phasentransferkatalysators, umsetzt.

Was die Menge des angewandten Hydroxids anbelangt, so setzt man zweckmäßigerweise etwa 200 bis etwa 500 Molprozent, vorzugsweise etwa 200 bis etwa 250 Molprozent Hydroxid pro auszutauschendem Halogenatom ein. Das Hydroxid kann sowohl als wäßrige Lösung als auch in wasserfreier Form zugegeben werden, wobei die Verwendung wäßriger Hydroxidlösungen wegen ihrer guten Zudosierbarkeit bevorzugt wird.

Die ggfs. eingesetzten Phasentransferkatalysatoren werden beispielsweise in einer Menge von etwa 0,5 bis etwa 30 Gew.-%, bezogen auf das Hydroxid, Zugesetzt. Derartige Phasentransferkatalysatoren sind beispielsweise in Dehmlow/Dehmlow "Phase Transfer Catalysis", Verlag Chemie, Weinheim 1983, beschrieben und sind in der Regel quartäre Ammonium- oder Phosphoniumverbindungen, sowie Kronenether oder Pyridiniumverbindungen. Als Beispiele hierfür seien Tetraalkylammoniumchlorid oder -bromid, Tetraalkylphosphoniumchlorid oder -bromid, Tetraphenylphosphoniumchlorid oder -bromid, Distearyldimethylammoniumcholorid oder -bromid, Hexadecyltributylphosphoniumchlorid oder -bromid, Pyridiniumchlorid oder -bromid oder 18-Krone-6 genannt. Neben einem einzelnen Phasentransferkatalysator können auch Gemische verschiedener Phasentransferkatalysatoren eingesetzt werden.

Als inerte organische Lösungsmittel kommen beim erfindungsgemäßen Verfahren beispielsweise Chlorbenzol, Dichlorbenzol, Trichlorbenzol, Xylol, Toluol, Chlortoluol oder N-Methylpyrrolidon in Frage. Neben organischen inerten Lösungsmitteln können auch Zweiphasensysteme aus Wasser und einem organischen inerten Lösungsmittel angewandt werden. Üblicherweise wird in einem Wasser/Lösungsmittelgemisch gearbeitet, wobei die Gewichtsverhältnisse von Wasser:Lösungsmittel 50:1 bis zu 1:20 betragen. Nach beendeter Umsetzung wird das Lösungsmittel durch azeotrope Destillation, eventuell unter Zugabe von Wasser abgetrennt. Es kann ohne Verlust in die nächste Umsetzung zurückgeführt werden.

Die Umsetzung kann drucklos oder unter Druck durchgeführt werden. Bei niedrigsiedenden Lösungsmitteln ist die Druckfahrweise vorteilhaft.

Die Reaktionszeiten bewegen sich zwischen etwa 2 und etwa 12 Stunden.

In der Deutschen Patentschrift 865 595 wird ein Verfahren zur Herstellung von Oxyarylketonen beschrieben, wobei Halogenbenzophenone mit Alkalien, Alkoholaten oder Phenolaten bei höheren Temperaturen umgesetzt werden. Im Falle der Umsetzung mit Ätznatron oder Ätzkali wird in wäßrigem Medium gearbeitet.

Die Erfindung wird durch die nachstehenden Beispiele näher erläutert, ohne darauf beschränkt zu werden.

### Beispiel 1

In einen Edelstahlautoklav werden 161,6 g (0,5 mol) 1,4-Bis-(4-fluorbenzoyl)-benzol, 83,7 g (2,05 mol) NaOH, 2500 g Wasser und 200 g Chlorbenzol vorgelegt und unter stetigem Rühren 7 Stunden auf 200° C erhitzt. Nach beendeter Umsetzung werden durch azeotrope Destillation 195 g Chlorbenzol abdestilliert. Die orange Lösung wird auf 50° C abgekühlt und mit 350 g HCl (ca. 30 %ig) auf pH = 2 gestellt. Das ausgefallene weiße 1,4-Bis-(4-hydroxybenzoyl)-benzol wird mit Wasser neutral gewaschen. Nach dem Trocknen werden 156 g (= 98,0 % d. Th.) mit einem Festpunkt von 318 - 319° C erhalten.

### Beispiel 2

In einem Edelstahlautoklav werden 177,6 g (0,5 mol) 1,4-Bis-(4-chlorbenzoyl)-benzol, 83,7 g (2,05 mol) NaOH, 2500 g Wasser und 200 g Chlorbenzol vorgelegt und unter stetigem Rühren 7 Stunden auf 220° C erhitzt. Nach beendeter Umsetzung werden durch azeotrope Destillation 190 g Chlorbenzol abdestilliert. Die orange Lösung wird auf 50° C abgekühlt und mit 350 g HCl (ca. 30 %ig) auf pH = 2 gestellt. Das ausgefallene weiße 1,4-Bis-(4-hydroxybenzoyl)-benzol wird mit Wasser neutral gewaschen. Nach dem Trocknen werden 154 g (= 96,8 % d. Th.) mit einem Festpunkt von 318 - 319° C erhalten.

## Patentansprüche

1. Verfahren zur Herstellung von
1,4-Bis-(4-hydroxybenzoyl)-benzol der Formel (I) 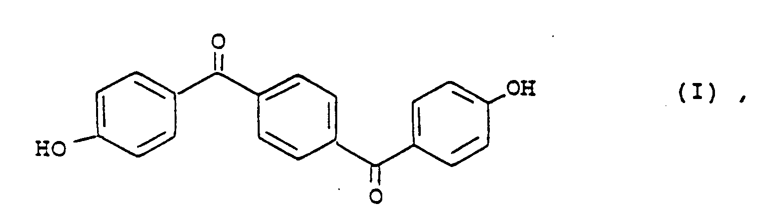 dadurch gekennzeichnet, daß man 1,4-Bis-(4-halogenbenzoyl)-benzol der Formel (II) 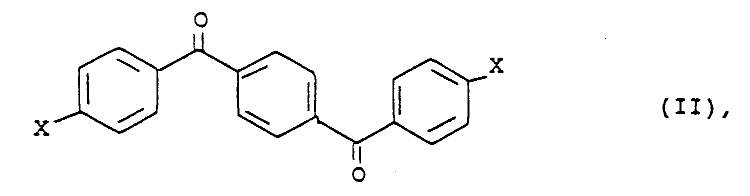 in welcher X ein Fluor-, Chlor- oder Bromatom bedeutet, mit einem Alkalimetallhydroxid oder Erdalkalimetallhydroxid oder mit Mischungen aus diesen Hydroxiden in einem gegenüber den Reaktionskomponenten bei den Reaktionsbedingungen inerten organischen Lösungsmittel oder in einem Zweiphasensystem aus Wasser und einem inerten organischen Lösungsmittel bei Temperaturen von etwa 100° C bis etwa 250° C, ggfs. in Gegenwart eines Phasentransferkatalysators, umsetzt.

2. Verfahren nach Anspruch 1, dadurch gekennzeichnet, daß man bei Temperaturen von etwa 150 bis etwa 220° C umsetzt.

3. Verfahren nach mindestens einem der Ansprüche 1 und 2, dadurch gekennzeichnet, daß man mit Lithium-, Natrium-, Kalium-, Calcium- oder Magnesiumhydroxid oder Mischungen daraus umsetzt.

4. Verfahren nach mindestens einem der Ansprüche 1 bis 3, dadurch gekennzeichnet, daß man mit etwa 200 bis etwa 500 Molprozent Alkali- oder Erdalkalimetallhydroxid pro auszutauschendem Halogenatom umsetzt.

5. Verfahren nach mindestens einem der Ansprüche 1 bis 4, dadurch gekennzeichnet, daß man mit etwa 200 bis etwa 250 Molprozent Alkali- oder Erdalkalimetallhydroxid pro auszutauschendem Halogenatom umsetzt.

6. Verfahren nach mindestens einem der Ansprüche 1 bis 5, dadurch gekennzeichnet, daß man in Chlorbenzol, Dichlorbenzol, Trichlorbenzol, Xylol, Toluol oder N-Methylpyrrolidon als inertem Lösungsmittel umsetzt.

7. Verfahren nach mindestens einem der Ansprüche 1 bis 5, dadurch gekennzeichnet, daß man in einem Zweiphasensystem aus Wasser und einem inerten organischen Lösungsmittel umsetzt.

8. Verfahren nach mindestens einem der Ansprüche 1 bis 5 und 7, dadurch gekennzeichnet, daß man in einem wäßrigen Zweiphasensystem aus Wasser und Chlorbenzol, Dichlorbenzol, Trichlorbenzol, Xylol, Toluol, Chlortoluol oder N-Methylpyrrolidon umsetzt.

9. Verfahren nach mindestens einem der Ansprüche 1 bis 8, dadurch gekennzeichnet, daß man in Gegenwart einer quartären Ammonium- oder Phosphoniumverbindung, einer Pyridiniumverbindung oder eines Kronenethers als Phasentransferkatalysator umsetzt.

10. Verfahren nach mindestens einem der Ansprüche 1 bis 9, dadurch gekennzeichnet, daß man in Gegenwart von Tetraalkylammoniumchlorid oder -bromid, Tetraalkylphosphoniumchlorid oder -bromid, Tetraphenylphosphoniumchlorid oder -bromid, Distearyldimethylammoniumcholorid oder -bromid, Hexadecyltributylphosphoniumchlorid oder -bromid, Pyridiniumchlorid oder -bromid oder 18-Krone-6 umsetzt.

## Claims

1. A process for the preparation of
1,4-bis(4-hydroxybenzoyl)benzene of the formula (I) 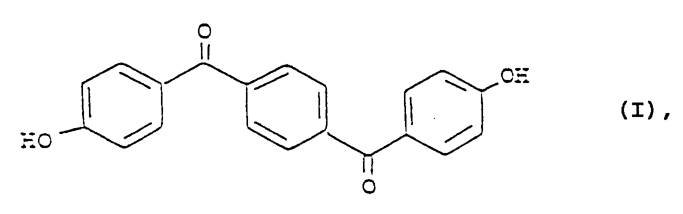 which comprises reacting a 1,4-bis(4-halobenzoyl)-benzene of the formula (II) 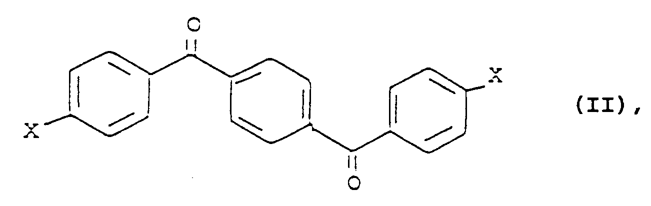 in which X is a fluorine, chlorine or bromine atom, with an alkali metal hydroxide or alkaline earth metal hydroxide or with mixtures of these hydroxides in an organic solvent which is inert to the reaction components under the reaction conditions or in a two-phase system composed of water and an inert organic solvent at temperatures of about 100°C to about 250°C, if appropriate in the presence of a phase transfer catalyst.

2. The process as claimed in claim 1, wherein the reaction is carried out at temperatures of about 150 to about 220°C.

3. The process as claimed in at least one of claims 1 and 2, wherein the reaction is carried out with lithium hydroxide, sodium hydroxide, potassium hydroxide, calcium hydroxide or magnesium hydroxide or mixtures thereof.

4. The process as claimed in at least one of claims 1 to 3, wherein the reaction is carried out with about 200 to about 500 mol percent of alkali metal hydroxide or alkaline earth metal hydroxide per halogen atom to be replaced.

5. The process as claimed in at least one of claims 1 to 4, wherein the reaction is carried out with about 200 to about 250 mol percent of alkali metal hydroxide or alkaline earth metal hydroxide per halogen atom to be replaced.

6. The process as claimed in at least one of claims 1 to 5, wherein the reaction is carried out in chlorobenzene, dichlorobenzene, trichlorobenzene, xylene, toluene or N-methylpyrrolidone as the inert solvent.

7. The process as claimed in at least one of claims 1 to 5, wherein the reaction is carried out in a two-phase system composed of water and an inert organic solvent.

8. The process as claimed in at least one of claims 1 to 5 and 7, wherein the reaction is carried out in an aqueous two-phase system composed of water and chlorobenzene, dichlorobenzene, trichlorobenzene, xylene, toluene, chlorotoluene or N-methylpyrrolidone.

9. The process as claimed in at least one of claims 1 to 8, wherein the reaction is carried out in the presence of a quaternary ammonium or phosphonium compound, a pyridinium compound or a crown ether as the phase transfer catalyst.

10. The process as claimed in at least one of claims 1 to 9, wherein the reaction is carried out in the presence of tetraalkylammonium chloride or bromide, tetraalkylphosphonium chloride or bromide, tetraphenylphosphonium chloride or bromide, distearyldimethylammonium chloride or bromide, hexadecyltributylphosphonium chloride or bromide, pyridinium chloride or bromide or 18-crown-6.

## Revendications

1. Procédé de préparation du 1,4-bis-(4-hydroxybenzoyl)-benzène de formule I 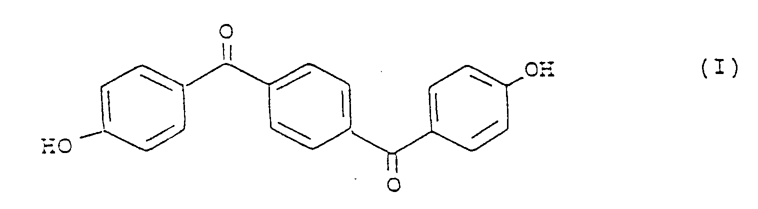 procédé caractérisé en ce que l'on fait réagir un 1,4-bis-(4-halogénobenzoyl)-benzène de formule II 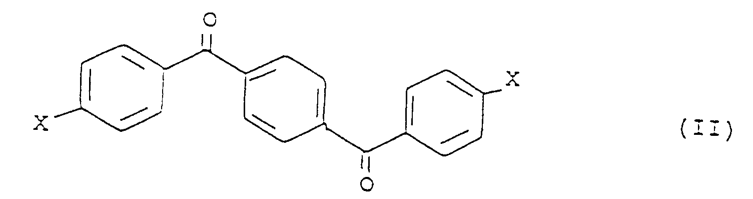 X désignant un atome de fluor, de chlore ou de brome, avec un hydroxyde de métal alcalin ou alcalino-terreux, ou plusieurs de ces hydroxydes à la fois, dans un solvant organique inerte à l'égard des composants réactionnels dans les conditions de la réaction ou dans un milieu à deux phases formé d'eau et d'un solvant organique inerte, à des températures d'environ 100 à 250°C, éventuellement en présence d'un catalyseur de transfert de phases.

2. Procédé selon la revendication 1, caractérisé en ce que l'on opère à des températures d'environ 150 à 220°C.

3. Procédé selon la revendication 1 et/ou 2, caractérisé en ce que l'on opère avec de l'hydroxyde de lithium, sodium, potassium, calcium ou magnésium ou avec plusieurs de ces hydroxydes à la fois.

4. Procédé selon une ou plusieurs des revendications 1 à 3, caractérisé en ce que l'on opère avec environ 200 à 500 Mol% de l'hydroxyde de métal alcalin ou alcalino-terreux par atome d'halogène à échanger.

5. Procédé selon une ou plusieurs des revendications 1 à 4, caractérisé en ce que l'on opère avec environ 200 à 250 Mol% de l'hydroxyde de métal alcalin ou alcalino-terreux par atome d'halogène à échanger.

6. Procédé selon une ou plusieurs des revendications 1 à 5, caractérisé en ce que l'on opère dans du chlorobenzène, du dichlorobenzène, du trichlorobenzène, du xylène, du toluène ou de la N-méthylpyrrolidone comme solvant inerte.

7. Procédé selon une ou plusieurs des revendications 1 à 5, caractérisé en ce que l'on opère dans un milieu à deux phases formé d'eau et d'un solvant organique inerte.

8. Procédé selon une ou plusieurs des revendications 1 à 5 et 7, caractérisé en ce que l'on opère dans un milieu aqueux à deux phases formé d'eau et de chlorobenzène, de dichlorobenzène, de trichlorobenzène, de xylènes, de toluène, de chlorotoluène ou de N-méthylpyrrolidone.

9. Procédé selon une ou plusieurs des revendications 1 à 8, caractérisé en ce que l'on opère en présence d'un composé d'ammonium ou de phosphonium quaternaire ou bien d'un composé de pyridinium ou d'un éther en couronne comme catalyseur de transfert de phases.

10. Procédé selon une ou plusieurs des revendications 1 à 9, caractérisé en ce que l'on opère en présence d'un chlorure ou bromure de tétraalkylammonium, de tétraalkylphosphonium, de tétraphénylphosphonium, de distéaryldiméthylammonium, d'hexadécyltributylphosphonium ou de pyridinium ou encore en présence d'une 18-couronne-6.
